# EUROPEAN PATENT APPLICATION

(11) **EP 2 801 620 A1**
(43) Date of publication of application: **12.11.2014**
(21) Application number: 12864065.3
(22) Date of filing: 14.11.2012
(51) Int. Cl.: C12Q 1/02, C12N 5/0735, C12N 5/10

(54) **DEVELOPMENT OF SIMPLE DISCRIMINATION METHOD FOR LOW-QUALITY ES CELLS AND iPS CELLS AS INDICATOR OF BIOLOGICAL CLOCK, AND DEVELOPMENT OF CELL EVALUATION METHOD AS INDICATOR OF BIOLOGICAL CLOCK**

(30) Priority: 06.01.2012 JP 2012001506
(71) Applicant: Kyoto Prefectural Public University Corporation, Kyoto-shi, Kyoto 6028566 (JP)
(72) Inventor: YAGITA, Kazuhiro, Kyoto-shi Kyoto 602-8566 (JP); UMEMURA, Yasuhiro, Kyoto-shi Kyoto 602-8566 (JP)
(74) Representative: Winter, Brandl, Fürniss, Hübner, Röss, Kaiser, Polte - Partnerschaft mbB
(86) International application number: PCT/JP2012/079552
(87) International publication number: WO 2013/103053

(57) **Abstract**

A present invention provides a method for evaluating pluripotent stem cells comprising: analyzing expression of a clock gene in cells differentiated from a pluripotent stem cell, and evaluating the pluripotent stem cells based on the degree of the expression.

## Description

### Technical Field

The invention relates to a method and a kit for evaluating pluripotent stem cells using a biological clock as an indicator.

### Background Art

The biggest problem in providing regenerative medicine using ES cells or iPS cells is the onset of a disease, such as cancer, derived from implanted cells. The greatest cause for this is said to be contamination of undifferentiated cells. In addition, problems caused by the low quality of ES cells or iPS cells used are pointed out. Thus, in achieving regenerative medicine by using cell implantation, quality control of ES cells and iPS cells is very important. Accordingly, researchers in Japan and overseas have developed various methods and have been making an effort to establish quality evaluation standards for iPS cells.

Non-patent Literature 1 discloses that ES cells and iPS cells do not have a biological clock and that a biological clock is developed by differentiation induction in vitro. However, this article does not contain data on evaluation of the quality of ES cells or iPS cells.

### Citation List

### Non-patent Literature

Non-patent Literature 1: K. Yagita et al., Proc. Natl. AcadSci. USA. February 23, 2010; 107(8) 3846-3851.

### Summary of Invention

### Technical Problem

An object of the invention is to provide a method and a kit for evaluating a pluripotent stem cell.

### Solution to Problem

Most living organisms on the earth have a biological clock (i.e., circadian clock) with a cycle of about 24 hours in order to know the time of day. Mammals have a biological clock in the cells of almost their entire body, and it is involved in control of cell function. In previous studies, the inventors found that pluripotent stem cells ("versatile cells") such as ES cells and iPS cells lack a biological clock and that a biological clock is developed when these cells are made differentiated.

When studying the mechanism of the development of the biological clock in the course of cell differentiation based on the above findings, the inventors found that the biological clock is not developed normally in pluripotent stem cells (e.g., ES cells) that have abnormal expression, such as a cancer gene, even if the cells are differentiated. The inventors thought that, by using this property and examining the ability for pluripotent stem cells used in regenerative medicine, such as iPS cells or ES cells, to develop a biological clock due to cell differentiation, it would be possible to exclude the cells that fail to develop a biological clock that at least a body cell has. The inventors thus addressed the establishment of such a method.

The inventors in this case developed a method for evaluating the quality of pluripotent stem cells including iPS cells and ES cells simply and quantitatively by using a biological clock as an indicator.

The invention provides the following methods and kits for evaluation.
Item 1. A method for evaluating pluripotent stem cells comprising: analyzing expression of a clock gene in cells differentiated from pluripotent stem cells, and evaluating the pluripotent stem cells based on the degree of the expression.
Item 2. The method according to Item 1, wherein the clock gene is Per2 and/or Bmal1 and/or Dbp.
Item 3. The method according to Item 1, wherein the pluripotent stem cells are ES cells or iPS cells.
Item 4. The method according to Item 1, further comprising introducing a gene construct including a reporter gene linked to a promoter of the clock gene, wherein the analyzing comprises analyzing an expression pattern of a clock gene based on expression of the reporter gene.
Item 5. The method according to Item 4, wherein the reporter gene is a fluorescent protein gene, a luciferase gene, a β-galactosidase gene, or a chloramphenicol acetyltransferase gene.
Item 6. A kit for evaluating pluripotent stem cells comprising a gene construct including a reporter gene linked to a promoter of a clock gene.
Item 7. The kit according to Item 6, wherein the clock gene is Per2 and/or Bmal1 and/or Dbp.
Item 8. The kit according to Item 6, wherein the pluripotent stem cells are ES cells or iPS cells.
Item 9. The kit according to Item 6, wherein the reporter gene is a fluorescent protein gene, a luciferase gene, a β-galactosidase gene, or a chloramphenicol acetyltransferase gene.

### Advantageous Effects of Invention

According to the invention, since a biological clock is not developed normally in pluripotent stem cells that have abnormal expression, such as a cancer gene, such pluripotent stem cells can be excluded from normal pluripotent stem cells. Thus, early selection and use of pluripotent stem cells that have no risk of becoming cancerous have become possible. Although a variety of studies have been conducted using pluripotent stem cells, it is expected that more appropriate studies will be conducted by selecting pluripotent stem cells that have no risk of becoming cancerous.

According to the invention, by examining the ability to develop a biological clock due to cell differentiation of pluripotent stem cells used in regenerative medicine, such as iPS cells and EP cells, it is possible to exclude the cells that fail to develop a biological clock that at least a body cell has. Thus, a method for simply and quantitatively evaluating the quality of pluripotent stem cells including iPS cells and ES cells was established.

### Brief Description of Drawings

Fig. 1: Fig. 1 shows oscillations of the biological clock that appears when differentiation of normal mice ES cells (a model of high-quality iPS cells) is induced according to the method of the invention.
Fig. 2: Fig. 2 shows oscillations of the biological clock that appears when differentiation of the ES cells characterized by weak expression of the cancer gene c-Myc (a model of low-quality ES/iPS cells) is induced according to the method of the invention, and shows frequency analysis with fast Fourier transform.
Fig. 3: Fig. 3 shows a quantitative evaluation of the ability for normal ES cells to develop a biological clock due to the differentiation induction culture. (A) Intensity of relative bioluminescence on day 7, day 14, day 21, day 28, and day 35 after the differentiation induction. (B) Relative power on day 7, day 14, day 21, day 28, and day 35. Higher relative power indicates clearer oscillation of the biological clock.
Fig. 4: Fig. 4 shows the intensity of bioluminescence of human iPS cells (hiPS) before differentiation and 4 weeks after in vitro differentiation induction. The formation of a circadian clock is reproduced by differentiation induction.

### Description of Embodiments

In the specification, clock genes include Clock, Bmal1, Period (Per1, Per2, Per3), Cryptochrome (Cry1, Cry2), Albumin D-box binding protein (Dbp), and the like. Preferred clock genes are Bmal1, Per2, and Dbp.

The method of the present invention enables analyzing expression of at least one clock gene. Analyzing expression includes measuring the expression amount of a clock gene. Measuring the expression amount of a clock gene can be performed by measuring the expression amount of a reporter gene expressibly linked to a promoter of the clock gene.

A pluripotent stem cell refers to a cell that can be differentiated into at least two types of cells, and that has a clock gene that stops or almost stops. Preferred pluripotent stem cells are ES cells and iPS cells, but other than these cells, any cells that can be divided into at least two types of cells, and that have a clock gene that stops or almost stops are included in the pluripotent stem cells to be evaluated in the invention. Since a clock gene functions in what are referred to as adult stem cells, these cells are not included in the pluripotent stem cells of the invention. Cells during direct reprogramming can be included in the pluripotent stem cells to be evaluated in the invention if the cells change into cells having greater differences in properties, as when the phylesis of the cells (endoderm, mesoderm, and ectoderm) change, and if a clock gene of the cells stops or almost stops.

Pluripotent stem cells may originate from a mammal such as a human, a monkey, a chimpanzee, a mouse, a rat, a rabbit, a goat, a dog, a cat, a cow, a horse, a pig, or the like.

The pluripotent stem cells used for the invention, such as ES cells and iPS cells, exist as a cell population. Since such cell populations have a uniform property (i.e., quality), an evaluation of all the pluripotent stem cells is possible by evaluating some of them according to the method of the invention.

Expression of the clock gene can be measured by measuring expression of mRNA by Northern blotting or a method using an antibody such as Western blotting or ELISA. Preferably, a reporter gene is linked downstream of either one of the promoters of a clock gene, and the expression amount of the reporter gene is measured to evaluate the expression of the clock gene. Reporter genes include genes such as fluorescent protein genes (e.g., GFP, CFP, BFP, YFP, and DsRED), luciferase genes (firefly luciferase, *Vargula hilgendorfii* luciferase, *Renilla* luciferase, and the like), β-galactosidase, and chloramphenicol acetyltransferase. Luciferase genes are preferred as a reporter gene.

In a preferred embodiment, the present invention enables evaluation of pluripotent stem cells by introducing a gene construct including a reporter gene linked to a promoter of the clock gene into the pluripotent stem cells to be evaluated or differentiated cells thereof, and by analyzing the expression amount of the reporter gene in the differentiated pluripotent stem cell.

A kit of the present invention includes the gene construct. The kit may further include at least one of the following: pluripotent stem cells such as ES cells and iPS cells, a differentiation induction medium, a culture medium for the pluripotent stem cell, and a culture vessel such as a Petri dish.

In the specification, "evaluation" of pluripotent stem cells means evaluation of the ability (i.e., potential) for the pluripotent stem cells to differentiate into the intended cells without differentiating into cells other than the intended cells, such as cancer cells. The higher the expression rate of the clock gene in cells differentiated from the pluripotent stem cells is, the greater the function of the pluripotent stem cells after being differentiated into the intended cells becomes. For example, as indicated in the Examples, when 92% or more, particularly when 100%, of the pluripotent stem cells express a clock gene about 4 weeks after the differentiation, it can be evaluated that the pluripotent stem cells become functional cells after the differentiation induction (i.e., evaluated as high). When 12% of the pluripotent stem cells express a clock gene 4 weeks after the differentiation, it can be determined that the pluripotent stem cells are less likely to become functional cells after the differentiation induction (i.e., evaluated as low). The higher the expression rate of the clock gene in cells differentiated from the pluripotent stem cells is, the higher the evaluation of the original pluripotent stem cells becomes.

For low-quality iPS cells in which a disorder of the biological clock is observed after differentiation, there is concern that such cells cause various disorders such as cancer after differentiation when implanted into mammals, including humans.

Recently, the relation between the biological clock and cancer has been attracting attention. In 2007, the World Health Organization (WHO) recognized "shiftwork that involves circadian disruption" as a risk factor of "Group 2A", which is probably carcinogenic. Currently, studies on cancer and the biological clock are being intensively carried out all over the world, and the fact that the biological clock is disrupted in some, but not all, the cancer cells is beginning to be reported (Shou-Jen Kuo et al., Virchows Arch (2009) 454: 467-474). The report that the disruption of the biological clock can be seen in cancer cells is a finding that supports the theory of the present invention that the development of the biological clock is disrupted in low-quality iPS cells. Pluripotent stem cells, the differentiated cells of which are proven to have no disorder in the biological clock according to the present invention, have no risk of becoming cancerous and have excellent functionality in the resultant differentiated cells. Accordingly, such pluripotent stem cells can be used preferably as an implant material that is implanted into the body after differentiation.

For the method of the invention, pluripotent stem cells are contained in a vessel such as a petri dish, cultured in an appropriate medium, and cultured for several weeks in a differentiation induction medium. The culture may be performed for three weeks or longer, preferably for about three to five weeks, and particularly for about four weeks. Then, expression of a clock gene is evaluated. If the culture period after the differentiation induction is five to six weeks or longer, the cells gradually become weak and expression of the clock gene begins to decrease. Thus, too long a period for culture is undesirable. The culture period is preferably shorter than 5 weeks. The cells become confluent when the culture period after differentiation induction is about three to four weeks. Culture after the differentiation induction can be performed while exchanging media. The frequency of media exchange is, for example, but not limited to, once every two days. The media may be exchanged once a day or at another frequency.

When a fluorescent protein such as GFP is used for a reporter gene, light can be radiated onto the cells that were differentiated from the pluripotent stem cells to detect a fluorescent light. When lusiferase is used, lusiferin can be used to measure the amount of emitted light from lusiferase. When another reporter gene is used, expression of the clock gene can be evaluated, depending on the reporter gene, by using an appropriate evaluation method of a product of the reporter gene.

Reproducibility of the experiment for developing the biological clock in a protocol of differentiation induction culture according to the invention is 85% or higher, 95% or higher, 98% or higher, 99% or higher, or 100%. Thus, low-quality pluripotent stem cells and high-quality pluripotent stem cells can be evaluated with good reproducibility.

As high-quality pluripotent stem cells, the Examples used ES cells to which the reporter gene (luciferase gene) for monitoring expression of a clock gene (Per2, Bmal1) is incorporated; as low-quality pluripotent stem cells, the Examples used ES cells to which c-Myc gene was introduced, in addition to the reporter gene (luciferase gene) for monitoring expression of a clock gene (Per2, Bmal1), and in which the ES cells showed about 1.3 times to about 4 times the expression amount of an endogenous c-Myc gene in wild-type ES cells. Among these, the low-quality ES cells that show 1.3 times the wild-type ES cells did not have a statistically significant difference compared with the wild-type ES cells in the measurement of the expression amount of the c-Myc gene by quantitative PCR, which is typically used for the evaluation of the quality of cells, and were indistinguishable from the wild-type ES cells in the screening study using gene expression analysis. According to the method of the invention, as a result repeatedly performing determination tests on low-quality ES cells by using ES cells in which the expression of the cancer gene c-Myc slightly remains and the risk of carcinogenesis is high, low-quality ES cells were successfully determined in all the tests.

According to the evaluation method of the invention, it is possible to correctly evaluate even the quality of low-quality pluripotent stem cells, which could not be screened using conventional methods. Thus, the present invention is characterized in that it enables achieving evaluation of the quality of pluripotent stem cells, which could not be screened with conventional methods.

### Examples

The invention will be described in detail below by way of examples.

### Example 1

### (1) Embryoid Body Formation

Mouse ES cells with feeder cells removed are diluted in a medium for fibroblasts at 2×10⁴ cells/mL and 100 µL is plated in each well of a low-adhesion round-bottom 96-well plate. The cells are cultured for 48 hours at 37°C with 5% CO₂.

### (2) Differentiation Culture of Embryoid Body

The embryoid body produced in the hanging drop culture is transferred to a 24-well plate and cultured in a medium for fibroblasts for 25 days at 37°C with 5% CO₂. Subculture is not performed during this culture. Media are exchanged once a day or every two days while varying the interval so that it is not constant. The biological clock is measured after 25 days in the differentiation induction medium.

The composition of the medium for fibroblasts is as follows.

DMEM (Nakarai Tesque) / 10% FBS, 1 mM sodium pyruvate (GibcoBRL), 0.1 mM MEM non-essential amino acids (Gibco BRL), 0.1 mM 2-mercaptoethanol (Sigma), 50 units/mL penicillin-streptomycin (Nakarai Tesque)

In both the low-quality mouse ES cells and the high-quality mouse ES cells used, a gene construct (reporter vector) in which a firefly luciferase gene was linked to the back of a promoter of a clock gene(Per2, Bmal1) was introduced into mouse ES cells. The reporter vector that was produced by using a routine method was used, and a gene that was cut out of the vector sold by Clontech Laboratories, Inc., was used as the luciferase gene.

Wild-type high-quality mouse ES cells and low-quality mouse cells were used as ES cells. The high-quality ES cells and the low-quality ES cells as described above were used.

When the wild-type mouse ES cells (a model of high-quality iPS cells) were evaluated in accordance with the method of the invention, the biological clock was developed normally and its oscillation was confirmed in 100% of the cases (results of 196 experiments).

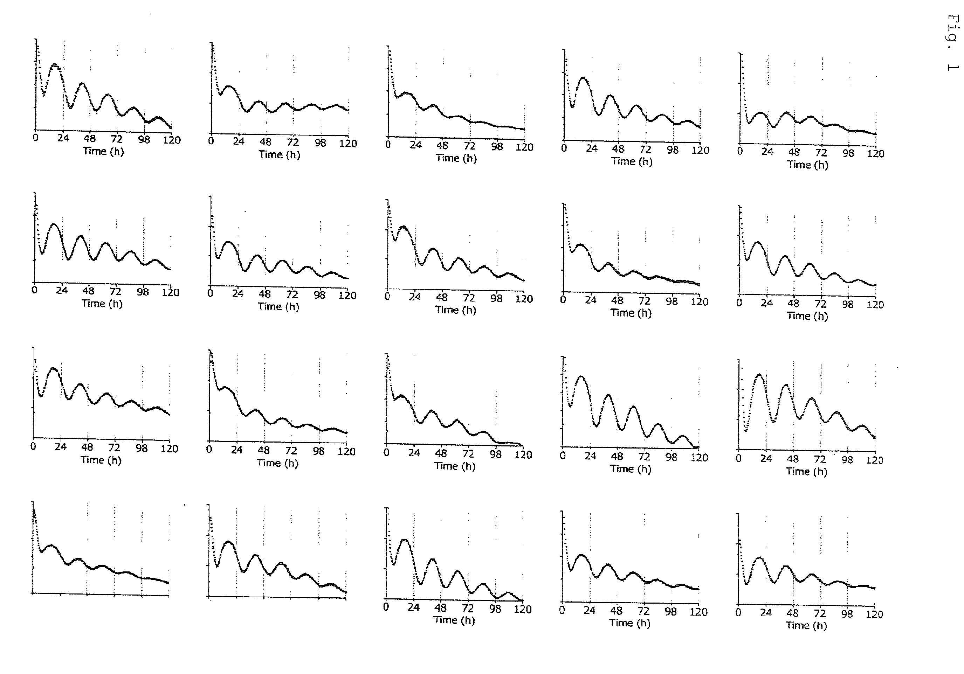

Fig. 1 shows raw data of experiments (20 times) in which measurement was actually performed.

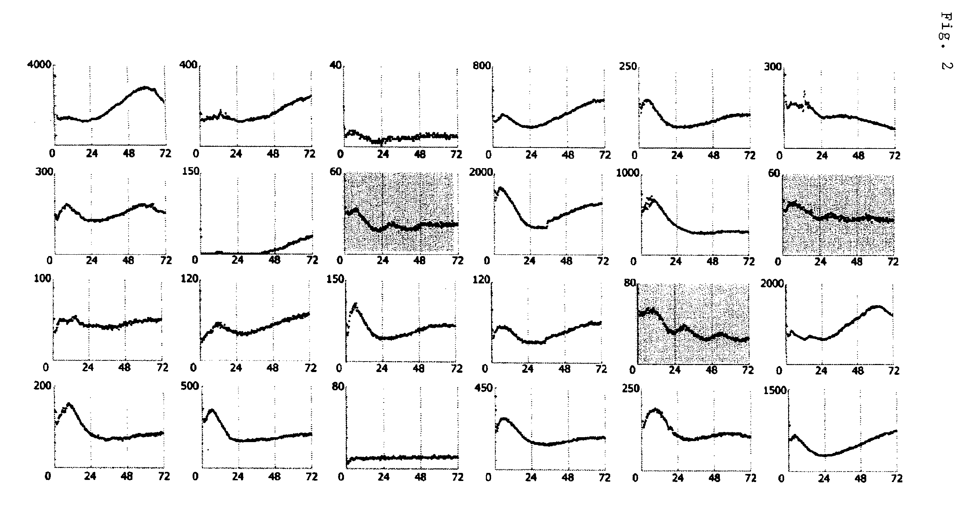

Fig. 2 is data of the development of the biological clock of the ES cells when the ES cells weakly and continuously expressing the cancer gene c-Myc, which is known as one of the four Yamanaka factors and used for producing an iPS cell, were established as a model of iPS cells and underwent differentiation induction culture according to the method of the invention as in Fig. 1. Unlike the case of the wild-type ES cells that were a model of the high-quality iPS cells, development of the biological clock was observed for only about 12% (indicated with red shading in Fig.2.)

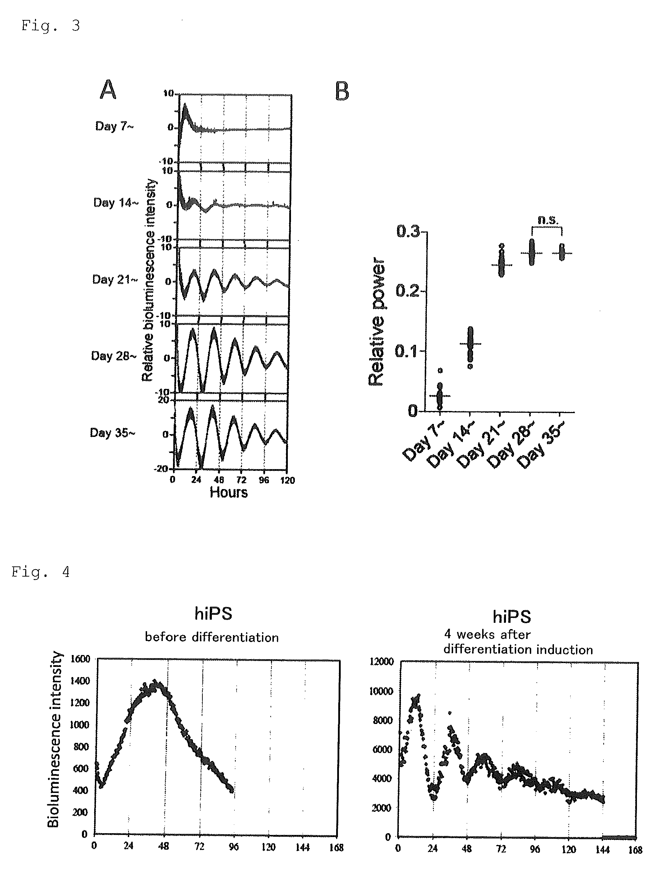

As a quantitative method for evaluating the presence of the development of the biological clock, fast Fourier transform (FFT) analysis was used to evaluate the strength of oscillations at a cycle frequency of about 24 hours (Fig. 3). By examining the expression of the clock gene after the differentiation induction in this manner, it became clear that it is possible to evaluate the quality of pluripotent stem cells (ES cells, iPS cells, or the like).

### Example 2

In order to evaluate the quality of human iPS cells, experiments were conducted to show that the in vitro cell evaluation method using a biological clock as indicator, which is a method developed by the present inventors, can be also actually applied to human iPS cells. When human iPS cells established at the Center for iPS Cell Research of Kyoto University underwent differentiation induction culture, the biological clock was normally formed four weeks after the differentiation induction (Fig. 4). This result strongly suggests that the method can also be used as a method for evaluating the quality of human iPS cells.

## Claims

1. A method for evaluating pluripotent stem cells comprising:
analyzing expression of a clock gene in cells differentiated from pluripotent stem cells; and
evaluating the pluripotent stem cells based on the degree of the expression.

2. The method according to claim 1, wherein the clock gene is Per2 and/or Bmal1 and/or Dbp.

3. The method according to claim 1, wherein the pluripotent stem cells are ES cells or iPS cells.

4. The method according to claim 1, further comprising introducing a gene construct including a reporter gene linked to a promoter of the clock gene into pluripotent stem cells to be evaluated, wherein the analyzing comprises analyzing an expression pattern of a clock gene based on expression of the reporter gene.

5. The method according to claim 4, wherein the reporter gene is a fluorescent protein gene, a luciferase gene, a β-galactosidase gene, or a chloramphenicol acetyltransferase gene.

6. A kit for evaluating pluripotent stem cells comprising a gene construct including a reporter gene linked to a promoter of a clock gene.

7. The kit according to claim 6, wherein the clock gene is Per2 and/or Bmal1 and/or Dbp.

8. The kit according to claim 6, wherein the pluripotent stems are ES cells or iPS cells.

9. The kit according to claim 6, wherein the reporter gene is a fluorescent protein gene, a luciferase gene, a β-galactosidase gene, or a chloramphenicol acetyltransferase gene.
